(19) Europäisches Patentamt
European Patent Office
Office européen des brevets

(11) Publication number: 0 224 257 A1

(12) # EUROPEAN PATENT APPLICATION

(21) Application number: 86116432.5

(22) Date of filing: 26.11.86

(51) Int. Cl.⁴: **C07D 235/30**, C07D 235/28, C07D 235/18, C07D 417/04, A61K 31/415, A61K 31/425

(30) Priority: 27.11.85 US 802508

(43) Date of publication of application: 03.06.87 Bulletin 87/23

(84) Designated Contracting States: AT BE CH DE ES FR GB GR IT LI LU NL SE

(71) Applicant: SYNTEX (U.S.A.) INC.
3401 Hillview Avenue P.O. Box 10850
Palo Alto California 94303(US)

(72) Inventor: Runkel, Richard Allen
741 Garland Drive
Palo Alto California 94303(US)

(74) Representative: Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) **Stabilized benzimidazole derivatives.**

(57) Amorphous, non-crystalline forms of benzimidazole derivatives have higher solubility and efficacy than crystalline forms. Novel compositions are disclosed which prevent crystallization of amorphous benzimidazole derivatives from either solid or liquid form.

EP 0 224 257 A1

# STABILIZED BENZIMIDAZOLE DERIVATIVES

This invention relates to amorphous benzimidazole derivatives which exhibit increased solubility and anthelmintic activity.

This invention also relates to a composition comprising an amorphous benzimidazole derivative and a stabilizing amount of a stabilizer polymer.

This invention also relates to a process for making the compounds and compositions of the invention, a method for stabilizing amorphous benzimidazole derivatives, a method for treating humans and other animals for helminthiasis and/or fascioliasis, and pharmaceutical and veterinary compositions therefor.

## Related Disclosures

Benzimidazole derivatives with anthelmintic activity have long been known in the art. See, e.g., U.S. Pat. Nos. 3,017,415, 3,480,642, 3,574,485, 3,657,267, 3,915,986, 3,929,821 and 3,993,682. However, conventional benzimidazole derivatives are rarely soluble to a practical degree in pharmaceutically acceptable carriers. Most conventional benzimidazole derivatives are nearly insoluble in water. This presents problems both for formulation and for bioavailability.

It has long been assumed, since helminths infest the digestive tracts of the animals to be treated, that the benzimidazole derivative administered as anthelmintics need not enter the bloodstream to a significant extent. Indeed, the bioavailability of benzimidazole derivatives is typically very low. The plasma uptake of benzimidazole derivatives administered to monogastric animals is usually 2% to 40% or less of the dose administered. The compound crystalline mebendazole is only 3 to 4% absorbed when administered to humans. Thus, doses as high as 1 g t.i.d. of crystalline mebendazole are required to treat conditions such as alveolar echinococcosis. It has now been found that the plasma concentration of a benzimidazole derivative is crucial to its efficacy, and that an increased plasma concentration correlates with increased anthelmintic activity. Other researchers have increased the initial plasma uptake of anthelmintic benzimidazole derivatives by wet-milling the powders to increase the surface area. (See for example EPO Application Ser. No. 147,767-A.) As a result, the plasma concentration of anthelmintic benzimidazole may initially be high, but will decrease rapidly to the level obtained with unmilled crystalline powders.

It has now been found that one may increase the solubility and efficacy of benzimidazole derivatives by preparing them in an amorphous, non-crystalline form. The increased solubility results in a relatively constant plasma concentration of benzimidazole derivative at a level much higher than that obtainable by increasing surface area, thus allowing one to decrease the dose and/or increase the effectiveness. This in turn allows one to treat strains of helminths resistant to the dose levels of anthelmintic benzimidazole derivatives presently administered. It has also been discovered that one may stabilize the amorphous form, (i.e., prevent conversion of amorphous benzimidazole derivatives to crystalline benzimidazole derivatives) by forming a homogeneous composition of amorphous benzimidazole derivative and a stabilizer polymer. Said compositions may be either solid (e.g., powders or wet cakes) or liquid. Amorphous compositions of the invention are effective at doses about 60% to 10% the effective dose of a crystalline benzimidazole derivative.

## DEFINITIONS

The terms used in this Specification and the appended Claims have the following definitions unless specified otherwise.

The term "amorphous" means substantially free of crystalline material wherein "substantially" means between 70% and 100% of the sample is amorphous, preferably between 80% and 100% of the sample is amorphous, more preferably between 90% and 100% of the sample is amorphous. Amorphous benzimidazole derivatives may be distinguished from crystalline forms by a number of methods, including differential scanning calorimetry, X-ray diffraction, microscopic examination, and dissolution comparison. For example, differential scanning calorimetry distinguishes amorphous oxfendazole from crystalline oxfendazole because amorphous oxfendazole exhibits an exotherm at its melting point, whereas crystalline oxfendazole exhibits an endotherm. X-ray diffractograms of crystalline oxfendazole characteristically show several large peaks between 5° and 25° (plotting intensity vs. 2$\theta$), whereas X-ray diffractions of amorphous oxfendazole exhibit small peaks, if any. Under microscopic examination with polarized light, crystalline

oxfendazole exhibits birefringence, whereas amorphous oxfendazole does not. Using a Hanson dissolution apparatus with a pH 5.5 buffer, one can demonstrate that amorphous oxfendazole is between 2 and 20 times more soluble than crystalline oxfendazole. The amount of crystalline matter in a sample of a benzimidazole derivative is measured by means known in the art, for example by X-ray diffraction. Thus, an X-ray diffractogram of an amorphous material will exhibit few if any peaks between 10° and 20° ($2\theta$). If the area of any peaks between 10° and 20° is less than 10% of the area below the baseline of the X-ray diffractogram of an amorphous material then the sample contains less than 10% crystalline material.

The term "rapid precipitation" means the precipitation of a solid from solution at a rate sufficient to avoid crystallization. "Rapid addition" refers to the addition of a solution or reagent at a high rate of speed. Generally, addition is rapid if the solution or reagent is added within 10 minutes, preferably within 1 minute.

The term "anthelmintic" refers to compounds which are useful for treating helminthiasis in animals.

The term" benzimidazole derivative" refers to compounds of formula 1:

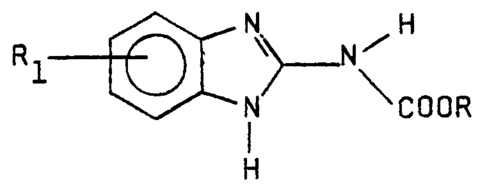

(1)

or a pharmaceutically acceptable salt thereof, wherein

R is lower alkyl; and

$R_1$ is $-S(O)_mR_2$, $-OR_2$, or

$Y_1(CH_2)_nY_2R_3$ where

$Y_1$ and $Y_2$ are each independently O, S, or S(O), $R_3$ is lower alkyl, phenyl, or naphthyl, and n is 1, 2, 3, or 4;

$R_2$ is lower alkyl, cycloalkyl, alkenyl of 3 to 7 carbon atoms, alkynyl of 3 to 7 carbon atoms, phenyl, benzyl, phenylethyl, or naphthyl; and

m is O or 1.

The term "oxfendazole" refers to the compound methyl 5-(phenylsulfinyl)-1H-benzimidazol-2-yl carbamate, also known as 5-phenylsulfinyl-2-carbomethoxyamino-benzimidazole.

The term "fenbendazole" refers to the compound methyl 5-(phenylthio)-1H-benzimidazol -2-yl carbamate, also known as 5-phenylthio-2-carbomethoxyamino-benzimidazole.

The term "RS-8852" refers to the compound methyl 5-(n-propylsulfinyl)-1H-benzimidazol-2-yl carbamate, also known as 5-n-propylsulfinyl-2-carbomethoxyaminobenzimidazole.

The term "albendazole" refers to the compound methyl 5-(n-propylthio)-1H-benzimidazol-2-yl carbamate, also known as 5-n-propylthio-2-carbomethoxyaminobenzimidazole.

The term "oxibendazole" refers to the compound methyl 5-(propoxy)-1H-benzimidazol-2-yl carbamate, also known as 5-propoxy-2-carbomethoxyaminobenzimidazole.

The term "animal" refers to both human and non-human species.

The term "pharmaceutical composition" refers to compositions for the treatment of human or domestic animals, including without limitation cattle, swine, dogs and sheep.

The term "veterinary composition" refers to compositions for the treatment of non-human domestic animals, including without limitation cattle, horses, swine, dogs, sheep, and the like.

The term "pharmaceutically acceptable salt" refers to salts of the subject compounds which possess the desired pharmacological activity and which are neither biologically nor otherwise undesirable. These salts are formed with inorganic acids such as hydrochloric acid, hydrobromic acid, sulfuric acid, nitric acid or phosphoric acid; or organic acids such as acetic acid, propionic acid, glycolic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid and the like. The term "pharmaceutically acceptable" includes all salts which are acceptable for human or veterinary use.

The term "effective amount" refers to the amount of benzimidazole derivative of formula 1 needed to effect treatment of a subject animal. The effective amount of an amorphous compound will vary from compound to compound. Generally, an effective amount of an amorphous compound of formula 1 will be between about 60% and 10% the effective amount of the compound in a crystalline form. An effective amount for anthelmintic use of compounds of formula 1 will generally be between about 0.02 mg/kg and 20 mg/kg. An effective amount of amorphous oxfendazole for cattle is generally between about 1 mg/kg and about 4 mg/kg, preferably between 2 mg/kg and 3 mg/kg, more preferably about 2.8 mg/kg. An effective

amount of amorphous oxfendazole for sheep is generally between about 1 mg/kg and about 4 mg/kg, preferably between 2 and 3.5 mg/kg, more preferably about 3 mg/kg. An effective amount of amorphous oxfendazole for swine is generally between about 2 mg/kg and about 8 mg/kg, preferably between 3 mg/kg and 5 mg/kg, more preferably about 4 mg/kg. An effective amount of amorphous oxfendazole for dogs is generally about 3 mg/kg.

The term "stabilizer polymer" refers to derivatives in compounds which are capable of preventing or inhibiting crystallization of benzimidazole derivatives. Stabilizer polymers include without limitation cellulosic derivatives, polyvinyl pyrrolidone and derivatives, xanthan gums, pectins, alginates, tragacanth and derivatives, Gum Arabic and derivatives, carrageenans, agar and derivatives, polysaccharides from microbial sources, arabinogalactans, galactomannans, dextrans and the like. Preferred stabilizer polymers are non-toxic to the subject animal at the level at which they are administered. Preferred stabilizer polymers are cellulosic derivatives and xanthan gums. More preferred stabilizer polymers are cellulosic derivatives.

The term " cellulosic derivative" refers to derivatives of cellulose which are capable of preventing or inhibiting the crystallization of benzimidazole derivatives. Preferred cellulosic derivatives are non-toxic to the subject animal at the level at which they are administered. Cellulosic derivatives include without limitation methyl cellulose, sodium carboxymethylcellulose, hydroxypropyl methylcellulose, hydroxypropyl cellulose, and the like. Preferred cellulosic derivatives are methyl cellulose, and sodium carboxymethylcellulose, especially methyl cellulose. Most preferred are Methocel ® A4C Premium, Methocel® A-15 Premium -(obtainable from Dow Chemical -USA), and sodium carboxymethylcellulose (CMC 7MF, obtainable from Hercules, Inc., Wilmington, Delaware).

The term "stable amorphous composition" refers to compositions containing an amorphous compound of formula 1 and a stabilizing amount of a stabilizer polymer. Thus, a stable amorphous composition is in a stable form, and resists the tendency to crystallize.

A "stabilizing amount" of a stabilizer polymer is the amount needed to prevent an amorphous compound of formula 1 from converting to a crystalline form. The stabilizing amount varies depending on the compound and the stabilizing polymer, but is between 0.0001 g/(g compound) and 0.9 g/(g compound), preferably between 0.001 g/(g compound) and 0.15 g/(g compound), more preferably between 0.02 g/(g compound) and 0.05 g/(g compound), and most preferably about 0.03 g/(g compound). It should be noted that the maximum amount of stabilizer polymer in the final pharmaceutical or veterinary composition is governed only by economic or practical necessities. As a practical matter, a maximum amount of stabilizer polymer will be the maximum amount that may be administered without injury to the subject. Thus, typical ratios (percentages) of stabilizer polymer to compound formula 1 needed to stabilize the compound of formula 1 include 0.001/1 (0.1%); 0.1/1 (10%); and 0.3/1 (30%).

The term "Keltrol®" refers to a xanthan gum which is commonly used in the pharmaceutical industry as a thickener. Keltrol ® is derived by fermentation of <u>Xanthomonas</u> <u>compestris</u>, and is available commercially from Kelco Co., San Diego, California. The preparation of Xanthan gums is described in U.S. Pat. Nos. 3,433,708 and 3,557,016, incorporated herein by reference.

The term "Carbopol" refers to an acrylic acid polymer resin with an average equivalent weight of about 76. Carbopol is commonly used in the pharmaceutical industry as a suspending agent, and is available commercially.

The term "Masonex" refers to a hemicellulose extract by-product of Masonite board process. Masonex is a viscous, dark-colored syrup commonly used in the pharmaceutical industry as a drug carrier, and is commercially available.

The term "treatment" as used herein covers any treatment of a disease in a mammal, and includes:

(i) inhibiting the disease, i.e., arresting its development; or

(ii) relieving the disease, i.e., causing regression of the disease.

The term "crumble" refers to a granulated mixture comprising an active ingredient, corn syrup, and common feed, which is pelletized and crushed to produce particles less than about 5 mm in diameter.

The term "alkyl" refers to a saturated hydrocarbon radical containing 1 to 20 carbon atoms. "Lower alkyl" refers to an alkyl radical of 1 to 6 carbons, for example, methyl, ethyl, n-propyl, i-propyl, butyl, s-butyl, t-butyl, pentyl, hexyl.

The term "cycloalkyl" refers to a cyclic saturated hydrocarbon radical containing 3 to 8 carbon atoms, for example, cyclopropyl, cyclobutyl, cyclopentyl, cyclohexyl, cycloheptyl, cyclooctyl.

The term "alkenyl" refers to a hydrocarbon radical of 3 to 7 carbon atoms, containing a double bond, for example, propenyl, allyl, butenyl, pentenyl, hexenyl, heptenyl.

The term "alkynyl" refers to a hydrocarbon radical of from 3 to 7 carbon atoms, containing a triple bond, for example, propynyl, propargyl, butynyl, pentynyl, hexynyl, heptynyl.

The term "alkoxy" refers to a radical of the form RO-, where R is lower alkyl or cycloalkyl as defined above.

The term "halo" refers to fluoro, chloro, bromo, or iodo.

One aspect of the invention is a benzimidazole derivative of formula 1 or a pharmaceutically acceptable salt thereof in an amorphous (non-crystalline) form:

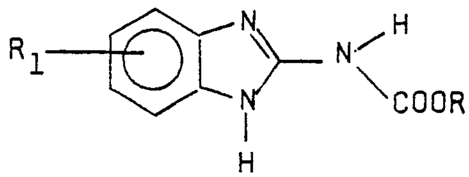

(1)

or a pharmaceutically acceptable salt thereof, wherein R is lower alkyl; and $R_1$ is $-S(O)_mR_2$, $-OR_2$, or $Y_1(CH_2)_{n}Y_2R_3$ where $Y_1$ and $Y_2$ are each independently O, S, or S(O), $R_3$ is lower alkyl, phenyl, or naphthyl, and n is 1, 2, 3 or 4; $R_2$ is lower alkyl, cycloalkyl, alkenyl of 3 to 7 carbon atoms, alkynyl of 3 to 7 carbon atoms, phenyl, benzyl, phenylethyl, or naphthyl; and m is 0 or 1.

Another aspect of the invention is a stable amorphous composition comprising a stabilizing amount of a stabilizer polymer and an amorphous compound of formula 1.

Another aspect of the invention is a process for producing amorphous benzimidazole derivatives, which process comprises precipitating a benzimidazole derivative of formula 1 from an acidic or basic solution by rapidly adding an amount of base or acid sufficient to adjust the solution to the pH of lowest benzimidazole solubility.

Another aspect of the invention is a process for producing stable amorphous compositions, which process comprises rapidly precipitating a benzimidazole derivative of formula 1 from an acidic or basic solution by adding a basic or acidic solution containing a stabilizing amount of a stabilizer polymer.

Another aspect of the invention is a process for producing stable amorphous compositions, which process comprises spray drying a benzimidazole derivative of formula 1, or a combination of benzimidazole derivatives of formula 1 from an acidic or basic solution containing a stabilizing amount of a stabilizer polymer.

Another aspect of the invention is a pharmaceutical or veterinary composition, which composition comprises a stabilizing amount of a stabilizer polymer; and an amorphous compound of formula 1, or a combination of amorphous compounds of formula 1.

Another aspect of the invention is a method of treating an animal for helminthiasis, which method comprises administering to a mammal in need thereof an effective amount of an amorphous composition of formula 1, or a combination of amorphous compositions of formula 1.

Another aspect of the invention is the use of an amorphous compound of formula, or a combination of amorphous compounds of formula to prepare a veterinary composition for the treatment of helminthiasis.

Another aspect of the invention is a process for preparing an amorphous compound of formula 1

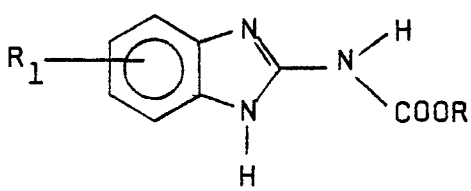

(1)

or a pharmaceutically acceptable salt thereof, wherein

R is lower alkyl; and

$R_1$ is $-S(O)_m R_2$, $-OR_2$, or $Y_1(CH_2)_nY_2R_3$ where

$Y_1$ and $Y_2$ are each independently O, S, or S(O), $R_3$ is lower alkyl, phenyl, or naphthyl, and n is 1, 2, 3, or 4;

$R_2$ is lower alkyl, cycloalkyl, alkenyl of 3 to 7 carbon atoms, alkynyl of 3 to 7 carbon atoms, phenyl, benzyl, phenylethyl, or naphthyl; and

m is 0 or 1;

which process comprises:

A. (a) dissolving a compound of formula 1 in a base or acid to dissolve or solubilize said compound of formula 1, followed by
(b) adding an amount of an acid or base under conditions to precipitate said compound of formula 1 in an amorphous form; or

B. (a) dissolving a compound of formula 1 in an acid or base to dissolve or solubilize said compound of formula 1, followed by
(b) spray drying the solution of step (a) to produce an amorphous compound of formula 1; or

C. (a) dissolving a compound of formula 1 in an acid or base solution containing a stabilizing amount of a stabilizer polymer, followed by
(b) spray drying the solution of step (a) to produce an amorphous compound of formula 1; or

D. (a) dissolving a compound of formula 1 in an acid or base within a first pH range to dissolve or solubilize said compound of formula 1, followed by
(b) adding a stabilizing amount of a stabilizer polymer in a solution of a second pH range opposite to that used in step (a), followed by
(c) adding acid of base to reestablish a third acid or base pH range on the same side of the pH scale as said first pH range to form an amorphous form of a compound of formula 1.

The first aspect of the invention is a non-crystalline, amorphous compound of formula 1

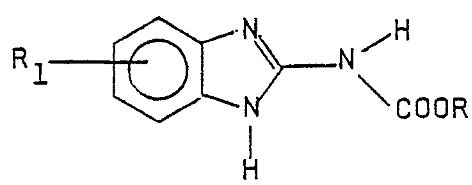

(1)

or a pharmaceutically acceptable salt thereof, wherein
R is lower alkyl; and
$R_1$ is $-S(O)_mR_2$, $-OR_2$, or $Y_1(CH_2)_nY_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkynyl of 3 to 7 carbon atoms, phenyl, benzyl, phenylethyl, or naphthyl; and
m is 0 or 1.

A preferred subgenus of compositions of the invention is the amorphous benzimidazole derivatives of formula 1 wherein $R_1$ is $-S(O)_mR_2$ and R is methyl, especially where m is 1. Preferred species are those wherein $R_2$ is ethyl, n-propyl, 2-butyl, or phenyl, especially n-propyl or phenyl. Another preferred class is that wherein $R_1$ is $-S(O)_mR_2$ where m is 1 and R is methyl. Preferred species are those wherein $R_2$ is n-propyl or phenyl. Another preferred class is that wherein $R_1$ is $OR_2$ and R is methyl. Preferred species are those wherein $R_2$ is n-propyl or phenyl. Another preferred class is that wherein $R_1$ is $-Y_1(CH_2)_nY_2R_3$ and R is methyl, especially where $Y_1$is S(O) and $Y_2$ is 0. Preferred species are those wherein $R_3$ is methyl, especially where n is 1 or 2.

Another aspect of the invention is a composition comprising a compound of formula 1 and a stabilizing amount of a stabilizer polymer. A preferred subgenus is that wherein said stabilizer polymer is a cellulosic derivative; a more preferred subgenus is that wherein said cellulose derivative is methyl cellulose. A preferred class is that wherein $R_1$ is $-S(O)_mR_2$ and R is methyl, especially where m is 1. Preferred species are those wherein $R_2$ is ethyl, n-propyl, 2-butyl, or phenyl, especially n-propyl or phenyl. Another preferred class is that wherein $R_1$ is $-S(O)_mR_2$ where m is 0 and R is methyl. Preferred species are those wherein $R_2$ is n-propyl or phenyl. Another preferred class is that wherein $R_1$ is $OR_2$ and R is methyl. Preferred species are those wherein $R_2$ is n-propyl or phenyl. Another preferred class is that wherein $R_1$ is $-Y_1(CH_2)_nY_2R_3$ and R is methyl, especially where $Y_1$ is S(O) and $Y_2$ is 0. Preferred species are those wherein $R_3$ is methyl, especially where n is 1 or 2.

Another aspect of the invention is a process for producing amorphous benzimidazole derivatives, which process comprises rapidly precipitating a compound of formula 1 from an acidic or basic solution having a first pH by adjusting the pH of said solution to a second pH at which the compound of formula 1 has lower solubility. A preferred class is the process wherein said second pH is about the pH of minimum solubility of the crystalline benzimidazole derivative selected (usually about 4 to 6), especially where said first pH is between 8 and 11. A preferred subclass is the process wherein said compound of formula 1 is first formed or dissolved in a solution with a pH lower than 4 (especially where said solution is acetic acid, propionic

acid, hydrochloride acid, phosphoric acid, formic acid or nitric acid; preferably concentrated formic acid or hydrochloric acid; more preferably formic acid), followed by addition of base to raise the pH to about 8 (especially where said base is an organic or inorganic base such as ammonium hydroxide, sodium hydroxide, potassium hydroxide, calcium hydroxide, barium hydroxide, sodium methylate, sodium ethylate, sodium hydride, triethylamine or pyridine; preferably ammonium hydroxide or sodium hydroxide in aqueous or methanolic solution; more preferably ammonium hydroxide or sodium hydroxide in aqueous solution), followed by addition of a second acid to lower the pH to about 5 (preferably where said second acid is the same acid as the first acid, especially where said second acid is formic acid). Another preferred subclass is the method in which said compound of formula 1 is first formed or dissolved in a solution with a pH higher than 7.5 (preferably where said solution is aqueous or methanolic organic or inorganic base such as ammonium hydroxide sodium hydroxide, potassium hydroxide, calcium hydroxide, barium hydroxide, sodium methylate, sodium ethylate, sodium hydride, triethylamine or pyridine; preferably ammonium hydroxide or sodium hydroxide in aqueous or methanolic solution; more preferably ammonium hydroxide or sodium hydroxide in aqueous solution), followed by addition of a concentrated acid to lower the pH to about 5 (especially where said acid is acetic acid, propionic acid, hydrochloric acid, phosphoric acid, formic acid or nitric acid; preferably concentrated formic acid or hydrochloric acid; more preferably formic acid).

Another aspect of the invention is a process for producing amorphous benzimidazole derivatives, which process comprises spray drying a compound of formula 1 from a solution, especially an acidic solution. A preferred class is the process wherein said acidic solution comprises acetic acid, propionic acid, hydrochloric acid, phosphoric acid, formic acid or nitric acid; preferably concentrated formic acid or hydrochloride acid; more preferably concentrated formic acid) and a compound of formula 1.

Another aspect of the invention is a process for producing stable amorphous benzimidazole compositions, which process comprises rapidly precipitating a compound of formula 1 and a stabilizing amount of a stabilizer polymer from an acidic or basic solution having a first pH by adjusting the pH of said solution to a second pH at which the compound of formula 1 has lower solubility. A preferred class is the process wherein said second pH is about about the pH of minimum solubility of the crystalline benzimidazole derivative selected (about 4 to 6), especially where said first pH is between 8 and 11. A preferred subclass is the process wherein said compound or formula 1 is first formed or dissolved in a solution with a pH lower than 4 (especially where said solution is concentrated formic acid or hydrochloric acid, particularly formic acid), followed by addition of base and a stabilizing amount of a stabilizer polymer to raise the pH to about 8 (especially where said base is aqueous or methanolic ammonium hydroxide or sodium hydroxide, preferably aqueous ammonium hydroxide or sodium hydroxide), followed by addition of a second acid to lower the pH to about 5 (especially where said second acid is hydrochloric or concentrated formic acid, preferably concentrated formic acid). Another preferred subclass is the method in which said compound of formula 1 is first formed or dissolved in a solution with a pH higher than 7.5 containing a stabilizing amount of a cellulose derivative (especially where said solution is aqueous or methanolic ammonium hydroxide or sodium hydroxide, preferably aqueous ammonium hydroxide or sodium hydroxide), followed by addition of an acid to lower the pH to about 5 (especially where said acid is concentrated formic acid).

Another aspect of the invention is a process for producing stable amorphous benzimidazole compositions, which process comprises spray drying a compound of formula 1 and a stabilizing amount of a stabilizer polymer from a solution, especially an acidic or basic solution, particularly an acidic solution. A preferred class is the process wherein said solution comprises concentrated formic acid, a stabilizing amount of a stabilizer polymer, and a compound of formula 1.

Another aspect of the invention is a process for stabilizing benzimidazole derivatives or combinations of benzimidazole derivatives in an amorphous form, which process comprises rapidly precipitating a compound of formula 1, or a combination of compounds of formula 1, from an acidic or basic solution by addition of a basic or acidic solution containing a stabilizing amount of a stabilizer polymer.

Another aspect of the invention is a method of treating an animal for helminthiasis, which method comprises administering to a mammal in need thereof an effective amount of a composition comprising:

a stabilizing amount of a stabilizer polymer; and

an amorphous compound of formula 1, or a combination of amorphous compounds of formula 1.

Another aspect of the invention is a pharmaceutical or veterinary composition for the treatment of helminthiasis, which composition comprises:

a stabilizing amount of a stabilizer polymer; and

an amorphous compound of formula 1, or a combination of amorphous compounds of formula 1.

Another aspect of the invention is the use of an amorphous compound of formula 1 to prepare a veterinary composition for the treatment of helminthiasis.

Compositions of the invention show increased solubility and efficacy over compositions containing crystalline benzimidazole derivatives.

Compositions of the invention may be tested by direct efficacy tests (wherein test animals are infected, administered the compound of the invention or neutral solvent, and necropsied to check for remaining parasites) or by blood plasma level demonstrations where the composition comprises individual compounds of known efficacy.

## ADMINISTRATION

Anthelmintic compositions of a compound of formula 1 are effective when administered to the digestive system of a subject. When the subject is a ruminant, it is preferred to administer the compositions of the invention intraruminally.

In view of the foregoing as well as in consideration of the degree of severity of the condition being treated, age of subject and so forth, the effective dosage in accordance herewith can vary over a wide range. Generally, a therapeutically effective amount for amorphous oxfendazole ranges from about 0.02 to about 20 mg/kg body weight. Other amorphous anthelmintic benzimidazole derivatives may have varying dosage ranges. Preferably, amorphous oxfendazole is administered intraruminally to cattle in dosages between 1.5 and 3 mg/kg, preferably about 2.8 mg/kg body weight. In other terms, for a 100 kg subject, a therapeutically effective amount in accordance herewith would be, in preferred embodiments from about 2 mg to about 2000 mg of amorphous oxfendazole per subject, and preferably between 150 mg to 300 mg, more preferably about 280 mg per subject.

Preferably, amorphous oxfendazole is administered orally to sheep in dosages between 2 and 3.5 mg/kg, preferably about 3 mg/kg body weight. In other terms, for a 30 kg subject, a therapeutically effective amount in accordance herewith would be, in preferred embodiments from about 60 mg to about 105 mg of amorphous oxfendazole per subject, and preferably about 90 mg per subject.

## PREPARATION OF THE INVENTION

Oxfendazole and similar benzimidazole derivatives, are available from commercial sources. Oxfendazole and similar benzimidazole derivatives may alternatively be made by the process described in U.S. Pat. No. 3,929,821, which is incorporated herein by reference.

Stabilizer polymers are commercially available. For example, methylcellulose may be purchased from Dow Chemical under the trademark Methocel® A-15 or Methocel® A4C. Hydroxypropylcellulose may be purchased from Dow Chemical under the trademark Methocel® E-15 or Methocel® E-5. Sodium carboxymethylcellulose may be obtained from Hercules, Inc., under the designation CMC 7MF.

Amorphous compounds of formula 1 can be prepared by a variety of methods, characterized by the fact that the compound of formula 1 is precipitated rapidly and is not allowed time to equilibrate and crystallize. In the following methods "dried" should be interpreted to mean drying either to a solid or to a wet cake. For example, a compound of formula 1 may be precipitated from solution by adding a solvent in which said compound does not dissolve or, preferably, by any of the following methods:

In the first method, a compound of formula 1 is dissolved or prepared in an acidic solution having a pH lower than about 3. Presently preferred solvents are concentrated HCl and formic acid; concentrated formic acid is most preferred. The amorphous compound of formula 1 can be precipitated directly from the resulting acidic solution by the rapid addition of base with stirring, however, due to the solubility characteristics of the compounds involved, it is preferred to first basify the solution of a pH of about 8, taking care not to induce precipitation. The acidic solution may be basified with any strong base (see above list), for example sodium hydroxide in water or alcohol (such as methanol), preferably sodium hydroxide in water. The resulting basic solution is then acidified until the pH of the solution is about the pH of minimum benzimidazole solubility, a pH of about 4 to 6. The basic solution may be acidified with any concentrated acid (see above list), preferably concentrated formic acid. The resulting solution is then stirred until amorphous compound of formula 1 aggregates and precipitates, whereupon it is collected, washed and dried. For example, crystalline oxfendazole (10 g) is dissolved in concentrated formic acid (95-97%, 20 mL). A solution of NaOH (30 g) in water (5 L) is then slowly added (e.g., at about 60 mL/minute) with high shear

mixing, taking care to avoid incorporating air or inducing precipitation. Concentrated formic acid (about 17 mL) is then added with stirring until the pH of the resulting solution reaches 4 to 5. The solution is stirred until amorphous oxfendazole precipitates. The precipitate is collected by vacuum filtration, washed with distilled water, and dried to yield amorphous oxfendazole.

In the second method, a compound of formula 1 is first dissolved in a basic aqueous or alcoholic solution. The base may be any strong base, such as sodium hydroxide, ammonium hydroxide, potassium hydroxide, and the like. The resulting basic solution is then acidified until the pH of the solution is about the pH of minimum benzimidazole solubility, a pH of about 4 to 5. The basic solution may be acidified with any concentrated acid, preferably concentrated formic acid. The resulting solution is then stirred until amorphous compound of formula 1 aggregates and precipitates, whereupon it is collected, washed and dried. For example crystalline oxfendazole (10 g) is dissolved in a solution of NaOH (30 g) in water (5 L). Concentrated formic acid is then added with stirring until the pH of the resulting solution reaches 4 to 5. The solution is stirred until amorphous oxfendazole precipitates. The precipitate is collected by vacuum filtration, washed with distilled water, and dried to yield amorphous oxfendazole.

In the third method, a compound of formula 1 is dissolved or prepared in an acidic solution having a pH lower than about 3. Presently preferred solvents are concentrated HCl and formic acid; concentrated formic acid is most preferred. The amorphous compound of formula 1 is then spray dried to produce an amorphous compound of formula 1. For example, crystalline oxfendazole (10 g) is dissolved in concentrated formic acid (95-97%, 20 mL) and spray dried to yield amorphous oxfendazole.

Stable amorphous compositions of the invention can be prepared by any of the following methods;

In the first method, a compound of formula 1 is dissolved or prepared in an acidic solution having a pH lower than about 3. Presently preferred solvents are concentrated hydrochloric acid, phosphoric acid, propionic acid, acetic acid and formic acid; concentrated formic acid is most preferred. The amorphous compound of formula 1 can be precipitated directly from the resulting acidic solution by the rapid addition of base with stirring, however, due to the solubility characteristics of the compounds involved, it is preferred to first basify the solution to a pH of about 8 using a solution containing a stabilizing amount of a stabilizer polymer such as a cellulosic derivative, taking care not to induce precipitation. The acidic solution may be basified with any strong base, for example ammonium hydroxide or sodium hydroxide in water or alcohol - (such as methanol), preferably ammonium hydroxide or sodium hydroxide in water. The stabilizer polymer is preferably a cellulosic derivative; preferably methylcellulose or sodium carboxymethylcellulose, particularly methylcellulose. The resulting basic solution is then acidified until the pH of the solution is about the pH of minimum benzimidazole solubility, a pH of about 4 to 5. The basic solution may be acidified with any concentrated acid, preferably concentrated formic acid. The resulting solution is then stirred until the amorphous composition precipitates and aggregates, whereupon it is collected, washed and dried. For example, crystalline oxfendazole (10 g) is dissolved in concentrated formic acid (95-97%, 20 mL). A solution of NaOH (30 g) and methylcellulose (0.02 g) in water (5 L) is then slowly added (e.g., at about 60 mL/minute) with high shear mixing, taking care to avoid incorporating air. Concentrated formic acid (about 17 mL) is then added with stirring until the pH of the resulting solution reaches 4 to 5. The solution is stirred until stabilized amorphous oxfendazole precipitates. The precipitate is collected by vacuum filtration, washed with distilled water, and dried to yield the stable amorphous composition of the invention.

In the second method, a compound of formula 1 is first dissolved in a basic aqueous or alcoholic solution containing a stabilizing amount of a stabilizer polymer. The base may be any strong base, such as ammonium hydroxide, sodium hydroxide, potassium hydroxide and the like. The stabilizer polymer is preferably a cellulosic derivative; more preferably methylcellulose or sodium carboxymethylcellulose, particularly methylcellulose. The resulting basic solution is then acidified until the pH of the solution is about the pH of minimum benzimidazole solubility, a pH of about 4 to 5. The basic solution may be acidified with any concentrated acid, preferably concentrated formic acid. The resulting solution is then stirred until the stabilized amorphous composition of the invention aggregates and precipitates, whereupon it is collected, washed and dried. For example, Crystalline oxfendazole (10 g) and Methocel® A4C (0.02 g) is dissolved in a solution of NaOH (30 g) in water (5 L). Concentrated formic acid is then added with stirring until the pH of the resulting solution reaches 4 to 5. The solution is stirred until the amorphous composition precipitates. The precipitate is collected by vacuum filtration, washed with distilled water, and dried to yield stabilized amorphous oxfendazole.

In the third method, a stabilizing amount of a stabilizer polymer and a compound of formula 1 are dissolved or prepared in an acidic solution having a pH lower than about 3. Presently preferred solvents are concentrated HCl and formic acid; concentrated formic acid is most preferred. The solution is then spray dried to produce a stabilized amorphous composition of the invention. For example, crystalline oxfendazole (10 g) and Methocel® A-15 (0.02 g) are dissolved in concentrated formic acid (95-97%, 20 mL) and spray dried to yield stabilized amorphous oxfendazole.

The methods described above may also be performed using mixtures of compounds of formula 1. These methods can be performed at ambient temperature and pressure.

The following specific description is given to enable those skilled in the art to more clearly understand and practice the invention. It should not be considered as a limitation upon the scope of the invention, but merely as being illustrative and representative thereof.

EXAMPLE 1

(A) Ten grams of crystalline oxfendazole is dissolved in 20 mL of formic acid (95-97%). A solution of 30 g NaOH in 5 L distilled water is then slowly added to the oxfendazole solution over a period of 90 minutes with high shear mixing, taking care to avoid air incorporation and precipitation. Concentrated formic acid is than added rapidly with stirring until the pH is between 4 and 5. The resulting solution is stirred until the amorphous oxfendazole aggregates, whereupon it is collected by vacuum filtration, washed with distilled water, and dried.

Samples of amorphous oxfendazole are prepared following the above procedure, and are finely ground. The diffractograms are obtained using a Nicolet Model 12 X-ray Diffractometer using CuK $\alpha$ radiation, scanning from 3° to 30° $2\theta$, step width = 0.05°, step time = 2 seconds. A sample planchet of 0.016 is used. The sample is placed on the planchet and smoothed with a glass slide. The number of counts above background is obtained via computer analysis. The total peak area between 10° and 20° $2\theta$ using amorphous oxfendazole is compared with the total peak area between 10° and 20° $2\theta$ obtained using crystalline oxfendazole. The peak area from amorphous oxfendazole is less than 10% of the peak area obtained from crystalline oxfendazole.

(B) Ten grams of crystalline RS-8852 is dissolved in 20 mL of formic acid (95-97%). A solution of 30 g NaOH in 5 L distilled water is then slowly added to the RS-8852 solution over a period of 90 minutes with high shear mixing, taking care to avoid air incorporation and precipitation. Concentrated formic acid is then added rapidly with stirring until the pH is between 4 and 5. The resulting solution is stirred until the amorphous RS-8852 aggregates, whereupon it is collected by vacuum filtration, washed with distilled water, and dried.

Samples of amorphous RS-8852 are prepared following the above procedure, and are finely ground. The diffractograms are obtained using a Nicolet Model 12 X-ray Diffractometer using CuK$\alpha$ radiation, scanning from 3° to 30° $2\theta$, step width = 0.05°, step time = 2 seconds. A sample planchet of 0.016 is used. The sample is placed on the planchet and smoothed with a glass slide. The number of counts above background is obtained via computer analysis. The total peak area between 10° and 20° $2\theta$ using amorphous RS-8852 is compared with the total peak area between 10° and 20° $2\theta$ obtained using crystalline RS-8852. The peak area from amorphous RS-8852 is less than 10% of the peak area obtained from crystalline RS-8852.

(C) Similarly, proceeding as in part A or B above but substituting albendazole, oxibendazole, fenbendazole or another benzimidazole derivative for oxfendazole, the corresponding amorphous compounds are produced, all of which show X-ray diffraction patterns with peak areas less than 10% of the peak area obtained from the crystalline compound.

EXAMPLE 2

(A) 67.94 grams of crystalline oxfendazole is dissolved in 95% formic acid. A solution of 203.9 grams of sodium hydroxide in 33.30 liters of distilled water and 676 ml of 5% methylcellulose is then slowly added with high shear mixing, taking care during both additions to avoid air incorporation or precipitation.
Concentrated formic acid is then added with stirring until the pH is between 4 and 5. The resulting solution is stirred until the amorphous oxfendazole aggregates, whereupon it is collected by vacuum filtration,

washed with distilled water (followed by a resuspension in 1% methylcellulose), centrifuged, and dried. The resulting amorphous oxfendazole with methylcellulose present showed less than 10% of the peak area of crystalline oxfendazole by the X-ray diffraction procedure in Example 1.

(B) Twenty grams of RS-8852 is dissolved in 20 ml of formic acid (95-97%). With good stirring the formic acid solution is slowly added to 1 liter of 5°C distilled water containing 70 grams of sodium hydroxide and 40 grams of methylcellulose. While still stirring the solution, concentrated formic acid is added until the pH is 4 to 5. The amorphous RS-8852 aggregates, whereupon it is collected by centrifugation at 5°C. The mother liquor is decanted and the amorphous RS-8852 is resuspended in 500 ml of 5°C 2% methylcellulose. The solution is again centrifuged to recover the amorphous RS-8852, which is washed and dried. The resulting amorphous RS-8852 with methylcellulose present showed less than 10% of the peak area of crystalline RS-8852 by the X-ray diffraction procedure in Example 1.

(C) Similarly, proceeding as in part A or B above but substituting fenbendazole, albendazole, oxibendazole, or another benzimidazole derivative for oxfendazole or RS-8852, the corresponding stabilized compounds are produced which show by X-ray diffraction less than 10% of the peak area of the corresponding crystalline compound by the procedure in Example 1.

## EXAMPLE 3

(A) Ten grams of oxfendazole is dissolved in 20 ml of concentrated formic acid (95-97%). The resulting solution if then spray dried using a conventional spray drying apparatus to yield amorphous oxfendazole showing less than 10% of the peak area of crystalline oxfendazole by the X-ray diffraction procedure of Example 1.

(B) Similarly, proceeding as in part A above but substituting RS-8852, albendazole, fenbendazole, oxibendazole or another benzimidazole derivative for oxfendazole, the corresponding spray dried amorphous compounds are produced which show less than 10% of the peak area of the corresponding crystalline compound by the X-ray diffraction procedure of Example 1.

## EXAMPLE 4

(A) Ten grams of oxfendazole and 0.02 grams of Methocel A-15 are dissolved in concentrated formic acid (95-97%). The resulting solution if then spray dried using a conventional spray drying apparatus to yield amorphous oxfendazole with Methocel A-15 present showing less than 10% of the peak area of crystalline oxfendazole by the X-ray diffraction procedure of Example 1.

(B) Similarly, proceeding as in part A above but substituting RS-8852, albendazole, fenbendazole, oxibendazole or another benzimidazole derivative for oxfendazole, the corresponding spray dried amorphous compounds with Methocel A-15 present are produced which show less than 10% of the peak area of the corresponding crystalline compound by the X-ray diffraction procedure of Example 1.

## EXAMPLE 5

(Injectable Formulation)

A formulation suitable for injection was prepared as follows:

amorphous oxfendazole 5.0 g
methyl cellulose 0.3 g
methyl paraben 0.01 %
propyl paraben 0.005 %
polyethylene glycol 4000 1.0 g
sterile water for injection qs 100.0 ml

The formulation was prepared as follows: The polyethylene glycol was dissolved in a small amount of water at 70°C. To this was added the oxfendazole (with or without stabilizer polymer), methyl paraben, and propyl paraben. The methyl cellulose was then dissolved in about 10 mL of water, and the resulting solution stirred into the amorphous oxfendazole solution and brought to volume with distilled water.

EXAMPLE 6

(Suspension Formulations)

(A) A formulation suitable for oral or intraruminal administration was prepared as follows:
amorphous oxfendazole 1.5 g
methyl cellulose 0.25 g
methyl paraben 0.01 %
propyl paraben 0.005 %
Keltrol® 1.5 g
polyethylene glycol 4000 2.0 g
Carbopol 1.5 g
water qs 100.0 ml
The formulation (with or without the presence of stabilizer polymer) was prepared as in Example 5 above, with the Keltrol® added in solution with the methyl cellulose.

(B) Another formulation suitable for oral or intraruminal administration was prepared as follows:
amorphous oxfendazole 5.0 g
methyl cellulose 0.4 g
methyl paraben 0.01 %
propyl paraben 0.005 %
Keltrol® 1.0 g
polyethylene glycol 4000 2.0 g
Carbopol 1.5 g
water 100.0 ml
The formulation (with or without the presence of stabilizer polymer) was prepared as in Example 5 above, with the Keltrol® and Carbopol added in solution with the methyl cellulose.

(C) Another formulation suitable for oral or intraruminal administration was prepared as follows:
amorphous oxfendazole 12.0 g
methyl cellulose 1.2 g
methyl paraben 0.01 %
propyl paraben 0.005 %
Keltrol® 1.0 g
polyethylene glycol 4000 1.0 g
Carbopol 1.0 g
water 100.0 ml
The formulation (with or without the presence of stabilizer polymer) was prepared as in part B above.

EXAMPLE 7

(Top Dress Formulations)

(A) A "crumble" formulation suitable for use as a top dress on animal feed was prepared as follows:
amorphous oxfendazole 50.0 mg
methyl cellulose 0.25 mg
lactose 5.0 g
corn syrup 0.5 g
The formulation was prepared as follows: the powdered amorphous oxfendazole (with or without the presence of stabilizer polymer), methyl cellulose, and lactose are blended together and granulated with the corn syrup. After drying, the granules are tabletted and the tablets crushed to particles less than 5 mm in diameter.

(B) Another formulation suitable for use as a top dress on animal feed was prepared as follows:
amorphous oxfendazole 2.5 g
methyl cellulose 5.0 mg
Masonex 5.0 g
wheat bran 0.7 g
The formulation was prepared as follows: methyl cellulose is dissolved in a minimum quantity of water and

the amorphous oxfendazole (with or without the presence of stabilizer polymer) is added. The resulting slurry is then mixed with Masonex, and then mixed with wheat bran. The resulting mixture is then dried in a trag drying oven.

(C) A suspension formulation suitable for use as a top dress on animal feed was prepared as follows:
amorphous oxfendazole 10.0 g
methyl cellulose 0.5 g
methyl paraben 0.01 %
propyl paraben 0.005 %
Keltrol® 0.1 %
polyethylene glycol 4000 2.0 g
Carbopol 2.0 g
water qs 100.0 ml
The formulation was prepared following the same procedure as in Example 6(A).

EXAMPLE 8

(Tablet Formulations)

(A) A tablet formulation for administration to dogs and similar mammals was prepared as follows:
amorphous oxfendazole 50.0 mg
methyl cellulose 0.1 mg
starch 15.0 mg
lactose 150.0 mg
magnesium stearate 0.2 mg
The formulation was prepared as follows: a paste is prepared using 7.5 mg of starch, the methyl cellulose, and a minimal quantity of water. The paste is then used to granulate the amorphous oxfendazole (with or without the presence of a stabilizer polymer), lactose, and remaining starch. The resulting granulate is then dried, blended with the magnesium stearate, and then tabletted.

(B) A tablet formulation suitable for administration to humans and similar mammals was prepared as follows:
amorphous oxfendazole 100.0 mg
methyl cellulose 0.2 mg
microcrystalline cellulose 150.0 mg
magnesium stearate 0.2 mg
The formulation was prepared as follows: the powders are blended together (with or without the presence of a stabilizer polymer) and tabletted without further processing.

(C) A tablet formulation suitable for administration to cattle and similar mammals was prepared as follows:
amorphous oxfendazole 2.5 g
methyl cellulose 10.0 mg
starch 0.3 g
lactose 50.0 mg
magnesium stearate 0.2 mg
The formulation was prepared as follows: a paste is prepared using 7.5 mg of starch, the methyl cellulose, and a minimal quantity of water. The paste is then used to granulate the amorphous oxfendazole (with or without the presence of a stabilizer polymer), lactose, and remaining starch. The resulting granulate is then dried, blended with the magnesium stearate, and then tabletted.

EXAMPLE 9

The following is an experimental procedure to demonstrate the efficacy of the invention.

(A) Fifteen (15) goats, preferably from a single source, known to be infected with nematode parasites as evidenced by positive fecal egg counts are used. The animals would be preferably of one sex. They will have been to be contaminated with goat parasite eggs and larvae, particularly Ostertagia spp. (hereinafter "Ost") and Trichostrongylus spp. (hereinafter "Tri")

Treatment Groups

1. -Control
2. -9 mg amorphous oxfendazole/kg
3. -15 mg oxfendazole/kg

Animals are then subjected to necropsy on day 6 post-treatment. Gastrointestinal tract, lungs, and liver are examined using standard parasitological techniques for nematode and trematode parasites.

| | Percent Efficacy | |
|---|---|---|
| Treatment | Ost | Tri |
| 15 mg oxfendazole/kg | 77% | 80% |
| 9 mg amor. oxfendazole/kg | 70% | 98% |

(B) Sheep carrying nematodes with varying degrees of resistance to oxfendazole were treated with either levamisole, oxfendazole, or amorphous oxfendazole in accordance with the above procedure.

| Treatment | Ost | Tri | Nem | Oes | Tr. o |
|---|---|---|---|---|---|
| Levamisole 8 mg/kg | 98% | 49% | 100% | 100% | 37% |
| Oxfendazole 5 mg/kg | 0% | 100% | 22% | 100% | 38% |
| Oxfendazole 10 mg/kg | 28% | 99.7% | 80% | 100% | 62% |
| Am. Oxfendazole 3 mg/kg | 55% | 100% | 57% | 100% | 9.5% |
| Am. Oxfendazole 6 mg/kg | 22% | 100% | 82% | 100% | 69% |

Ost = Ostertagia spp.
Tri = Trichostrongylus spp.
Nem = Nematodirus spp.
Oes = Oesophagostomum spp.
Tr.o = Trichuris ovis

The above two studies suggest that amorphous oxfendazole is approximately equivalent in efficacy to oxfendazole, but at a reduced dose of only about 60% of that required for oxfendazole. No toxicity was seen at the above dosage levels.

EXAMPLE 10

The following is an experimental procedure to demonstrate blood plasma levels of the active compounds administered.

Dogs were administered orally either amorphous or crystalline oxfendazole at a dose of 10 mg/kg.

Blood samples were drawn and analyzed before administration and on days 1, 2, 3, 7, 10 and 13 following administration. The area under the curve was calculated for plasma concentration vs time profile. Dogs treated with amorphous oxfendazole evinced a higher plasma level of oxfendazole that dogs treated with oxfendazole in commercially available form.

| | Area Under the Curve (ng/ml hr) | |
|---|---|---|
| Dog No. | Amorphous Ox. | Crystalline Ox. |
| A | 20465.0 | 2700.0 |
| B | 9717.5 | 2622.0 |
| C | 11592.5 | 2130.0 |
| D | 24245.0 | 3341.0 |
| E | 12653.8 | 3107.5 |
| F | 25475.0 | 4149.5 |
| mean (n = 6) | 17358.1 | 3008.4 |
| +/- S.D. | 6880.7 | 698.4 |

A similar experiment with sheep showed similar increases in the area under the plasma concentration curve for amorphous oxfendazole over crystalline oxfendazole.

## Claims

1. An amorphous compound of formula 1

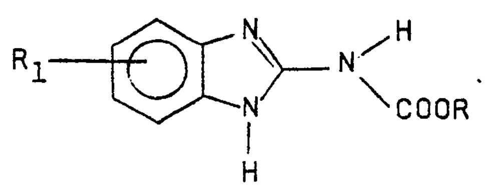

(1)

or a pharmaceutically acceptable salt thereof, wherein
R is lower alkyl;
$R_1$ is $-S(O)_mR_2$, $-OR_2$, or $Y_1(CH_2)_nY_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkynyl of 3 to 7 carbon atoms, phenyl, benzyl, phenylethyl, or naphthyl; and
m is 0 or 1.

2. The amorphous compound of Claim 1 wherein R is methyl.
3. The amorphous compound of Claim 2 wherein $R_1$ is $-S(O)_mR_2$.
4. The amorphous compound of Claim 3 wherein $R_2$ is phenyl or n-propyl.
The amorphous compound of Claim 4 wherein m is 1.
6. The amorphous compound of Claim 4 wherein m is 0.
7. The amorphous compound of Claim 5 wherein $R_2$ is phenyl, said compound is oxfendazole.
8. The amorphous compound of Claim 6 wherein $R_2$ is phenyl, said compound is fenbendazole.
9. The amorphous compound of Claim 5 or 6 wherein $R_2$ is n-propyl, said compound is RS-8852 or albendazole.
10. The amorphous compound of Claim 2 wherein $R_1$ is $OR_2$.
11. The amorphous compound of Claim 10 wherein $R_2$ is n-propyl, said compound is oxibendazole.
12. A stable amorphous composition comprising a stabilizing amount of a stabilizer polymer; and an amorphous compound of formula 1

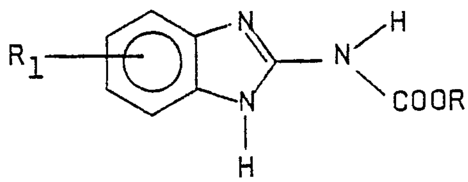

(1)

or a pharmaceutically acceptable salt thereof, wherein

R is lower alkyl;

$R_1$ is $-S(O)_mR_2$, $-OR_2$, or $Y_1(CH_2)_nY_2R_3$ where

$Y_1$ and $Y_2$ are each independently O, S, or S(O), $R_3$ is lower alkyl, phenyl, or naphthyl, and n is 1, 2, 3, or 4;

$R_2$ is lower alkyl, cycloalkyl, alkenyl of 3 to 7 carbon atoms, alkenyl of 3 to 7 carbon atoms, phenyl, benzyl, phenylethyl, or naphthyl; and

m is 0 or 1

13. The composition of Claim 12 wherein said amorphous compound of formula 1 is oxfendazole, fenbendazole, albendazole, 5-n-propylsulfinyl-2-carbomethoxyaminobenzimidazole, or oxibendazole.

14. The amorphous composition of Claim 13 wherein said stabilizer polymer comprises cellulose derivatives, polyvinyl pyrrolidone and derivatives, xanthan gums, pectins, alginates, tragacanth and derivatives, gum arabic and derivatives, carrageenans, agar and derivatives, polysaccharides from microbial sources, arabinogalactans, galactomannans, and dextrans.

15. The amorphous composition of Claim 14 wherein said stabilizer polymer is a cellulose derivative such as methylcellulose, Methocel®A-15, Methocel®A4C, or sodium carboxymethylcellulose.

16. A pharmaceutical or veterinary composition which comprises:

a carrier acceptable for pharmaceutical or veterinary use; and

an amorphous compound of formula 1, or a combination of amorphous compounds of formula 1

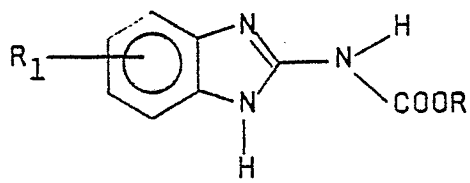

(1)

or a pharmaceutically acceptable salt thereof, wherein

R is lower alkyl; and

$R_1$ is $-S(O)_mR_2$, $-OR_2$, or $Y_1(CH_2)_nY_2R_3$ where

$Y_1$ and $Y_2$ are each independently O, S, or S(O), $R_3$ is lower alkyl, phenyl, or naphthyl, and n is 1, 2, 3, or 4;

$R_2$ is lower alkyl, cycloalkyl, alkenyl of 3 to 7 carbon atoms, alkynyl of 3 to 7 carbon atoms, phenyl, benzyl, phenylethyl, or naphthyl; and

m is 0 or 1.

17. The composition of Claim 16 wherein a stabilizing amount of a stabilizer polymer is added.

18. The composition of Claim 17 wherein said stabilizer polymer comprises cellulosic derivatives, polyvinyl pyrrolidone and derivatives, xanthan gums, pectins, alginates, tragacanth and derivatives, gum arabic and derivatives, carrageenans, agar and derivatives, polysaccharides from microbial sources, arabinogalactans, galactomannans, and dextrans.

19. The composition of Claim 20 wherein said stabilizer polymer is a cellulosic derivative such as methylcellulose, Methocel®A-15, Methocel®A4C, or sodium carboxymethylcellulose.

20. The composition of any one of Claims 16 to 19 wherein said compound of formula 1 is oxfendazole, fenbendazole, albendazole, 5-n-propylthio-2-carbomethoxyaminobenzimidazole, or oxibendazole.

21. The composition of any of Claims 17 to 20 which comprises:

0.001%-50% stabilizer polymer;

1%-99% carrier acceptable for pharmaceutical or veterinary use;

1%-95% amorphous compound of formula 1

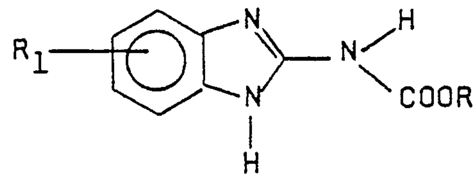

(1)

or a pharmaceutically acceptable salt thereof, wherein
R is lower alkyl; and
$R_1$ is $-S(O)_mR_2$, $-OR_2$, or $Y_1(CH_2)_nY_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkynyl of 3 to 7 carbon atoms, phenyl, benzyl, phenylethyl, or naphthyl; and
m is 0 or 1.

22. The composition of Claim 21 wherein said amorphous compound of formula 1 and said cellulosic derivative are present in a ratio between about 1000:1 and about 1:90, preferably between about 100:1 and about 5:1.

23. The composition of Claim 21 which is suitable for oral administration, or for parenteral or intraruminal injection, wherein
said amorphous compound of formula 1 is present in an amount between about 1% and about 40% by weight;
said stabilizer polymer is present in an amount between about 0.02% and about 3% by weight; and
said carrier acceptable for pharmaceutical or veterinary use is a liquid present in an amount between about 99% and about 57% by weight.

24. The composition of Claim 21 which is suitable for oral administration as a tablet or capsule, wherein
said amorphous compound of formula 1 is present in an amount between about 15% and about 98% by weight;
said stabilizer polymer is present in an amount between about 0.01% and about 85% by weight; and
said carrier acceptable for pharmaceutical or veterinary use is a solid present in an amount between about 85% and about 1% by weight.

The use of an amorphous compound of formula 1

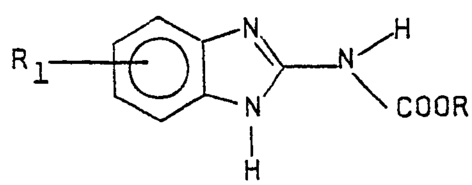

(1)

or a pharmaceutically acceptable salt thereof, wherein
R is lower alkyl; and
$R_1$ is $-S(O)_m R_2$, $-OR_2$, or $Y_1(CH_{2n}Y_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkynyl of 3 to 7 carbon atoms, phenyl, benzyl, phenylethyl, or naphthyl; and
m is 0 or 1.
to prepare a pharmaceutical or veterinary composition for the treatment of helminthiasis.

26. The use of a stabilizer polymer with an amorphous compound of formula 1

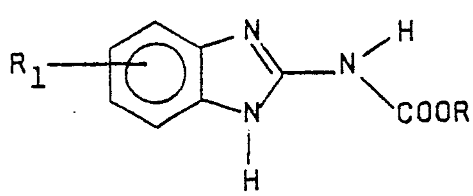

(1)

or a pharmaceutically acceptable salt thereof, wherein

R is lower alkyl; and
$R_1$ is $-S(O)_mR_2$, $-OR_2$, or $Y_1(CH_2)_nY_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkynyl of 3 to 7 carbon atoms, phenyl, benzyl, phenylethyl, or naphthyl; and
m is 0 or 1
to prepare a pharmaceutical or veterinary composition for the treatment of helminthiasis.

27. A process for preparing an amorphous compound for formula 1

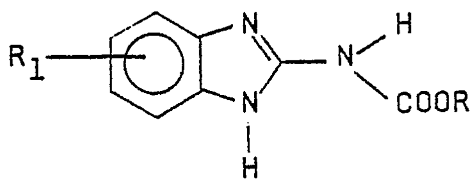

(1)

or a pharmaceutically acceptable salt thereof, wherein
R is lower alkyl; and
$R_1$ is $-S(O)_mR_2$, $-OR_2$, or $Y_1(CH_2)_nY_2R_3$ where
$Y_1$ and $Y_2$ are each independently O, S, or S(O), $R_3$ is lower alkyl, phenyl, or naphthyl, and n is 1, 2, 3, or 4;
$R_2$ is lower alkyl, cycloalkyl, alkenyl of 3 to 7 carbon atoms, alkenyl of 3 to 7 carbon atoms, phenyl, benzyl, phenylethyl, or naphthyl; and
m is 0 or 1;
which process comprises:

A. (a) dissolving a compound of formula 1 in a base or acid to dissolve or solubilize said compound of formula 1, followed by
(b) adding an amount of an acid or base under conditions to precipitate said compound of formula 1 in an amorphous form; or

B. (a) dissolving a compound of formula 1 in an acid or base to dissolve or solubilize said compound of formula 1, followed by
(b) spray drying the solution of step (a) to produce an amorphous compound of formula 1; or

C. (a) dissolving a compound of formula 1 in an acid or base solution containing a stabilizing amount of a stabilizer polymer, followed by
(b) spray drying the solution of step (a) to produce an amorphous compound of formula 1; or

D. (a) dissolving a compound of formula 1 in an acid or base within a first pH range to dissolve or solubilize said compound of formula 1, followed by
(b) adding a stabilizing amount of a stabilizer polymer in a solution of a second pH range opposite to that used in step (a), followed by
(c) adding acid of base to reestablish a third acid or base pH range on the same side of the pH scale as said first pH range to form an amorphous form of a compound of formula 1.

28. The process of Claim 27 wherein said acid comprises concentrated acetic acid, propionic acid, hydrochloric acid, phosphoric acid, formic acid, and nitric acid.

29. The process of Claim 27 or 28 wherein the pH range of step A(a), B(a), C(a), or D(a) is lower than about 3.

30. The process of Claim 27 or 28 wherein the pH range of step A(a), B(a), C(a), or D(a) is between about 8 and 10.

31. The process of Claim 27 or 28 where the pH range of step A(b), or D(c) is between about 4 and 6.

32. The process of any one of Claims 27 to 31 wherein said base comprises ammonium hydroxide, sodium hydroxide, potassium hydroxide, calcium hydroxide, barium hydroxide, sodium methylate, sodium ethylate, sodium hydride, triethylamine and pyridine.

33. The process of any one of Claims 27 to 32 wherein said stabilizer polymer comprises cellulosic derivatives, polyvinyl pyrrolidone and derivatives, xanthan gums, pectins, alginates, tragacanth and derivatives, gum arabic and derivatives, carrageenans, agar and derivatives, polysaccharides from microbial sources, arabinogalactans, galactomannans, and dextrans.

34. The process of Claim 33 wherein said stabilizer polymer is a cellulosic derivative such as methylcellulose, sodium carboxymethylcellulose, hydroxypropylmethylcellulose, and hydroxypropylcellulose.

European Patent Office

# EUROPEAN SEARCH REPORT

Application number

EP 86116432.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE - A1 - 3 347 579 (HOECHST AG)<br>* Claims; pages 4,5 *<br>-- | 1,12, 16,25, 27 | C 07 D 235/30<br>C 07 D 235/28<br>C 07 D 235/18<br>C 07 D 417/04<br>A 61 K 31/415<br>A 61 K 31/425 |
| A | CHEMCIAL ABSTRACTS, vol. 93, no. 6, August 11, 1980, Columbus, Ohio, USA<br>FREY,H.H.;KELLER, H. "Bioavailability of benzimidazole anthelmintics, from different pharmaceutical preparations in the horse"<br>page 456, column 1, abstract no. 53 873p<br>& Prakt. Tierarzt 1980, 61(3), 234, 239-40<br>-- | 1,12, 16,25, 27 | |
| A | EP - A1 - 0 131 762 (SUMITOMO CORP.)<br>* Claims *<br>---- | 1,12, 16,25, 27 | TECHNICAL FIELDS SEARCHED (Int. Cl.4)<br>C 07 D 235/00<br>C 07 D 417/00 |

The present search report has been drawn up for all claims

| Place of search | Date of completion of the search | Examiner |
|---|---|---|
| VIENNA | 02-03-1987 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document